# EUROPEAN PATENT APPLICATION

(11) **EP 0 697 223 A2**
(43) Date of publication of application: **21.02.1996**
(21) Application number: 95830251.5
(22) Date of filing: 19.06.1995
(51) Int. Cl.: A61M 15/00

(54) **Pocket-sized nebuliser for delivering drugs to the oral cavity**

(30) Priority: 20.07.1994 IT MI940533 U
(71) Applicant: PLASTIAPE S.p.A., I-22058 Osnago (Como) (IT)
(72) Inventor: Citterio, Gian Franco, c/o Plastiape S.p.A., I-22058 - Osnago (Como) (IT)
(74) Representative: Cicogna, Franco

(57) **Abstract**

The present invention relates to a pocket delivery device, specifically designed for delivering nebulized drug substances into the oral cavity, comprising a body into the inside of which can be introduced a bottle provided with a delivering or metering valve.

With said body there is associated a push-button provided with a delivering rod.

There are moreover provided a bottom element to be applied for closing the bottom of said body, and a cap which can be superimposed on said body at least at the region thereof affected by said push- button and delivering rod.

## Description

### BACKGROUND OF THE INVENTION

The present invention relates to a pocket delivery device specifically designed for delivering or metering nebulized drug substances into the oral cavity.

There are already known on the market delivering or metering devices which are provided with a spout element allowing a nebulized drug substance to be introduced directly into the oral cavity.

However, these prior devices have a comparatively large size and comprise a delivering spout which can not be protected, since no covering element is provided for protecting the metering spout as it is not used.

Thus, no pocket drug substance delivering device is available in the prior art, since no means has been provided for protecting from dirt the metering spout of such a device.

### SUMMARY OF THE INVENTION

Accordingly, the aim of the present invention is to solve the above mentioned problem, by providing a pocket delivery device, the metering or delivering spout thereof can be properly protected, so as to allow the device to be easily carried in a pocket without any danger of polluting by dirt.

Within the scope of the above mentioned aim, a main object of the present invention is to provide such a drug delivery device which can be constructed with a very reduced size.

Another object of the present invention is to provide such a drug delivery device which can be easily assembled by using automatic assembling machines, thereby allowing said device to be made on an industrial scale.

Yet another object of the present invention is to provide such a pocket drug delivery device which is very reliable and safe in operation.

According to one aspect of the present invention, the above mentioned aim and object, as well as yet other objects, which will become more apparent hereinafter, are achieved by a pocket delivery device, specifically designed for introducing into the oral cavity nebulized drug substances, characterized in that said device comprises a body, into the inside of which can be introduced a bottle having a delivering valve, with said body a push-button being associated, including a delivering rod, a bottom element to be applied to the bottom of said body for closing said bottom and a cap which can be superimposed on said body, at least at the region thereof affected by said bush-button and rod, being moreover provided.

### BRIEF DESCRIPTION OF THE DRAWINGS

Further characteristics and advantages of the invention will become more apparent hereinafter from the following detailed disclosure of a pocket delivery device, which is illustrated, by way of an indicative, but not limitative example, in the figures of the accompanying drawings, where:

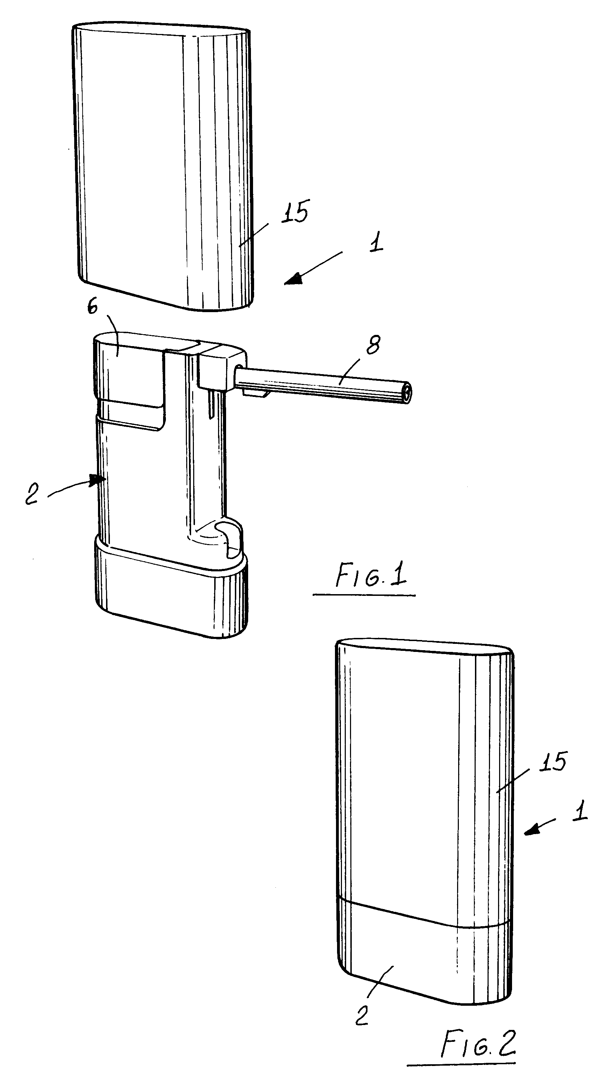

Figure 1 is a schematic perspective view illustrating the delivery device according to the present invention, the cap thereof being represented in an exploded form;
Figure 2 illustrates the pocket delivery device, at a closure position thereof and by a perspective view;

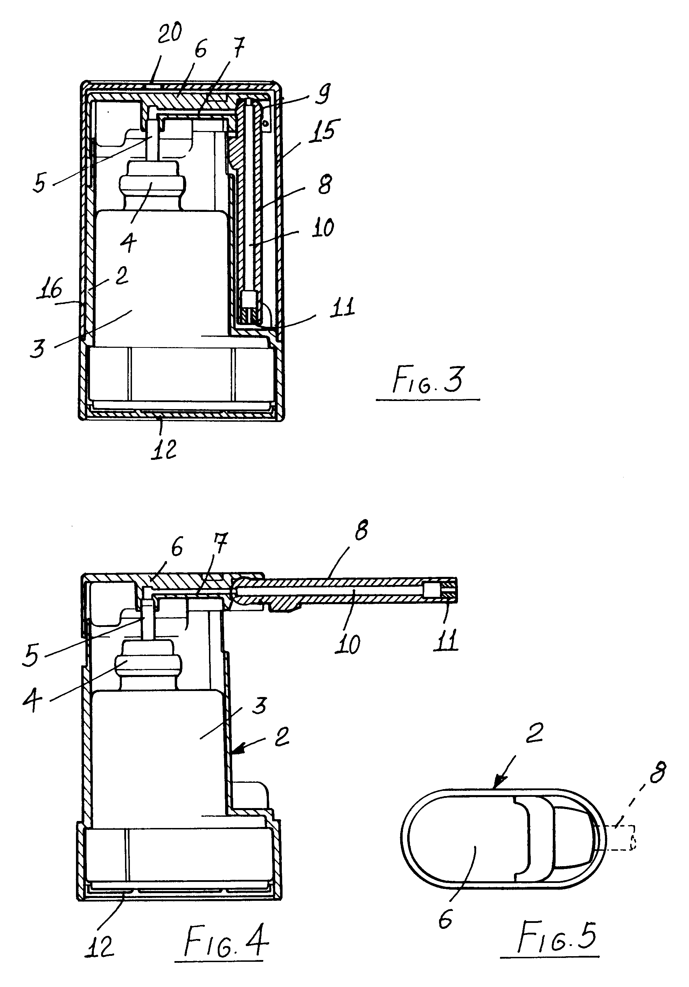

Figure 3 is a cross-sectional view illustrating the pocket delivery device in a closure condition thereof;
Figure 4 is a further cross-sectional view illustrating the pocket delivery device according to the invention in an open position thereof; and
Figure 5 is a top plan view illustrating the delivery device according to the present invention with the cap thereof being removed.

### DESCRIPTION OF THE PREFERRED EMBODIMENTS

With reference to the number references of the above mentioned figures, the pocket delivery device, which has been specifically designed for introducing into the oral cavity nebulized drug substances, and which has been generally indicated by the reference number 1, comprises a body 2, which has a substantially flat configuration and defining an inside hollow in which can be housed a bottle 3, including a delivering or metering valve 4, provided with a delivering small tube 5.

At the top portion of the body 3 is provided a push-button 6, which is slidably housed above the body 2 and is provided with a delivering or metering duct 7, fixedly engaging at the top end portion of the delivery tube 5.

At the end of the delivery duct 7 it is provided a delivering rod 8 which comprises, at the connection end portion thereof, a ball shaped enlarged portion 9, allowing the delivering rod 8 to be folded near the body 2, in a recess, which has been specifically provided, and which defines moreover, at a closure condition thereof, a shutter element preventing the communication with the delivering or metering duct 7.

Inside the delivering rod 8 is provided a core 10 which ends, and the free end portion of the delivering rod 8, with a pad 11.

The body 2 is closed, at the bottom thereof, by a bottom element 12 which can be applied by pressure and holds the bottle in its intended position.

The device comprises moreover a cap 15, the size of which is designed for superimposing on the body 2, at least at the region thereof affected by the push-button 6 and delivering rod 8, and which is arranged flush with the base of the body 2 owing to the provision of cut-outs 16 on the side surface of said body 2.

With the disclosed arrangement, a pocket delivery device is provided, in which the delivering or metering rod is always protected, as the device is not used, by the cap 15.

Moreover, the cap 15 will also operate to protect the delivering rod 8, as the delivery device is arranged in a cabinet.

Another important feature of the present invention is that the delivery device can be assembled by automatic assembling machines.

To that end, it is possible to provide a first embodiment in which the body 2, push-button 6, delivering rod 8, core 10 and pad 11 can be pre-assembled, and in which, after having applied the bottle and related delivering valve 4, it is possible to apply the closure bottom element 12 and cap 15.

According to another embodiment, it is possible to pre-assemble the body 2, push-button 6, delivering rod 8, core 9, bottle 10 as well as the cap 15.

The latter, in this case, will be provided with a throughgoing hole 20, arranged at the metering or delivering tube 5, in order to allow the introduction of an abutment element for the push-button 6, which will be easily fixedly assembled on the tube 5 during the assembling operation.

From the above disclosure it should be apparent that the invention fully achieves the intended aim and objects.

In particular, the fact is to be pointed out that a delivery device has been provided which can be easily carried in a pocket, since it has a flat configuration and, moreover, is specifically designed for protecting the portions thereof provided for contacting a person oral cavity, since the cap thereof will fully cover the delivering or metering rod as well as the push-button to which said rod is connected.

In practicing the invention, the used materials, provided that they are compatible to the intended use, as well as the contingent size and shapes, can be any, depending on requirements.

## Claims

1. A pocket delivery device, specifically designed for introducing into the oral cavity nebulized drug substances, characterized in that said device comprises a body, into the inside of which can be introduced a bottle having a delivering valve, with said body a push-button being associated, including a delivering rod, a bottom element to be applied to the bottom of said body for closing said bottom and a cap which can be superimposed on said body, at least at the region thereof affected by said bush-button and rod, being moreover provided.

2. A delivery device, according to Claim 1, characterized in that said delivering or metering rod is articulated to said push-button by a ball-shape enlarged portion provided for operating, as the delivering rod is folded on the body, as a shutter element for shutting-off the delivery of said drug substance.

3. A delivery device, according to Claims 1 and 2, characterized in that said device further comprises, inside said delivering or metering rod, a core element ending at the bottom with a pad arranged at an end portion of said delivering rod.

4. A delivery device, according to one or more of the preceding claims, characterized in that, in order to assemble said delivery device by automatic assembling machine, said body, push-button, delivering rod, core and pad are pre-assembled, whereas said bottle, bottom element and cap are applied subsequently.

5. A delivery device, according to one or more of the preceding claims, characterized in that, in order to assemble said delivery device on automatic assembling machines, said body, push-button, delivering rod, core, pad and cap are pre-assembled, said cap being provided with a throughgoing hole arranged at the delivering tube of the delivering or metering valve.
